Europäisches Patentamt

European Patent Office

Office européen des brevets

(19)

(11) Publication number: **0 397 419**
**A2**

(12) **EUROPEAN PATENT APPLICATION**

(21) Application number: **90304889.0**

(22) Date of filing: **04.05.90**

(51) Int. Cl.5: **C12P 21/08, C12N 5/12,**
**G01N 33/574, A61K 39/395,**
**C07K 15/00**

(30) Priority: **10.05.89 US 350651**

(43) Date of publication of application:
**14.11.90 Bulletin 90/46**

(84) Designated Contracting States:
**AT BE CH DE DK ES FR GB GR IT LI LU NL SE**

(71) Applicant: **HYBRITECH INCORPORATED**
**11095 Torreyana Road**
**San Diego California 92126(US)**

(72) Inventor: **Grauer, Lana Suzanne**
**13252 Caminito Pt. Del Mar**
**Del Mar, California 92014(US)**
Inventor: **Koda, Joy Elaine**
**10264 Meadowview Drive**
**San Diego, California 92131(US)**
Inventor: **Leung, Julia Pauline**
**7924 Camino Jonata**
**San Diego, California 92122(US)**

(74) Representative: **Hudson, Christopher Mark et**
**al**
**Erl Wood Manor**
**Windlesham Surrey GU20 6PH(GB)**

(54) **Novel tumor-associated antigen, antibodies, compositions and uses therefor.**

(57) An antigen, characterized by its reactivity to monoclonal antibody CCK061 and antibody CCR086, is a glycoprotein having a molecular weight in the range of about 600,000 to about 1,000,000 daltons and isoelectric points in the ranges of about 4.3 to about 4.5 and about 4.8 to about 5.0. Antibodies directed against the antigen, methods for their production and diagnostic and therapeutic uses therefor are provided.

EP 0 397 419 A2

## NOVEL TUMOR-ASSOCIATED ANTIGEN, ANTIBODIES, COMPOSITIONS AND USES THEREFOR

The present invention relates to the characterization of antigens, particularly tumor-associated antigens. In another aspect, it relates to antibodies having specific reactivity with such antigens. In yet another aspect, it relates to methods for producing such antibodies as well as diagnostic and therapeutic uses therefor.

The potential role of monoclonal antibodies in the diagnosis and treatment of cancer has been the focus of much recent investigation and speculation. Of particular interest are their use in immunoassays to detect and monitor the course of the disease, for example, during therapy. Also of particular interest are the potential applications of monoclonal antibodies for tumor imaging and therapy due to their capacity to bind to tumor-associated antigens in vivo.

Developments in monoclonal antibody technology have also made it possible to investigate the antigenic complexity of human tumors. Specifically, the precise immunoreactivity of monoclonal antibodies permits the identification and differentiation of distinct antigens expressed by human tumors. The characterization of such distinct tumor-associated antigens, therefore, provides a means to more fully exploit the production and use of monoclonal antibodies for cancer diagnosis and therapy.

Certain antigens are expressed by both human tumor cells and normal cells. These antigens are accordingly referred to not as "tumor specific" but as "tumor-associated" antigens. The diagnostic and therapeutic value of such tumor-associated antigens results from the excess quantity of antigen expressed by tumor cells relative to normal cells and the in vivo selectivity of antibodies for antigens expressed by tumor cells over normal cells. The relative selectivity of antibodies administered in vivo for antigen expressed by tumor cells is believed to result from: (1) the increased expression of antigen by cancerous cells due to the altered and rapid metabolism of malignant growth; and, (2) the increased accessibility of antigens expressed by tumor tissue to antibodies due to the breaKdown of barrier properties of the tumor cell membranes.

To date, only a limited number of tumor-associated antigens are well characterized. For example, Koprowski et al. in U.S. Patent No. 4,471,057, issued on September 11, 1984 and entitled "Detection of Colorectal Carcinoma," describe a colorectal carcinoma monosialoganglioside antigen reactive with a monoclonal antibody (hereinafter referred to as ZCE063, Centacor 19.9 or 19.9) expressed by the hybridoma cell line ATCC #HB 0859. According to Koprowski et al., the antigen is a glycolipid of endodermal origin. Another tumor-associated antigen (TAG-72, found on more than 80% of colon carcinoma, is described in Johnson et al., Cancer Research 46, 850-857 (1986). TAG-72 is reported to be a heavily glycosylated glycoprotein of high molecular weight that is reactive with monoclonal antibody B72.3 (hereinafter also referred to as ZBC062).

Additionally, certain tumor-associated antigens useful as diagnostic or prognostic markers may not be present in all patients or during all stages and manifestations of the disease. Diagnostic discrimination and therapeutic efficacy are, therefore, enhanced by the identification and characterization of more than one tumor-associated antigen expressed by the same tumor tissue. Furthermore, for purposes of cancer diagnosis and therapy, it is desirable to rely on a set or panel of distinct antigens associated with specific types of human tumors. Accordingly, there exists a need for further identification and characterization of unique tumor-associated antigens.

The present invention is predicated upon the discovery and characterization of a novel antigen present in human tissue and cancerous human cell lines. Accordingly, the invention is directed to a distinct tumor-associated glycoprotein antigen having a molecular weight in the range of about 600 Kd to about 1,000 Kd, with isoelectric points in the ranges of about 4.3 to about 4.5, and about 4.8 to about 5.0.

In accordance with the present invention, antibodies having specificity for the antigen defined herein and methods for the production of such antibodies are also provided. Additionally, the invention is directed to the use of such antibodies for the in vitro detection and diagnosis of cancer by immunohistochemical and immunoassay methods and for the in vivo diagnosis and treatment of cancer in humans as set forth below.

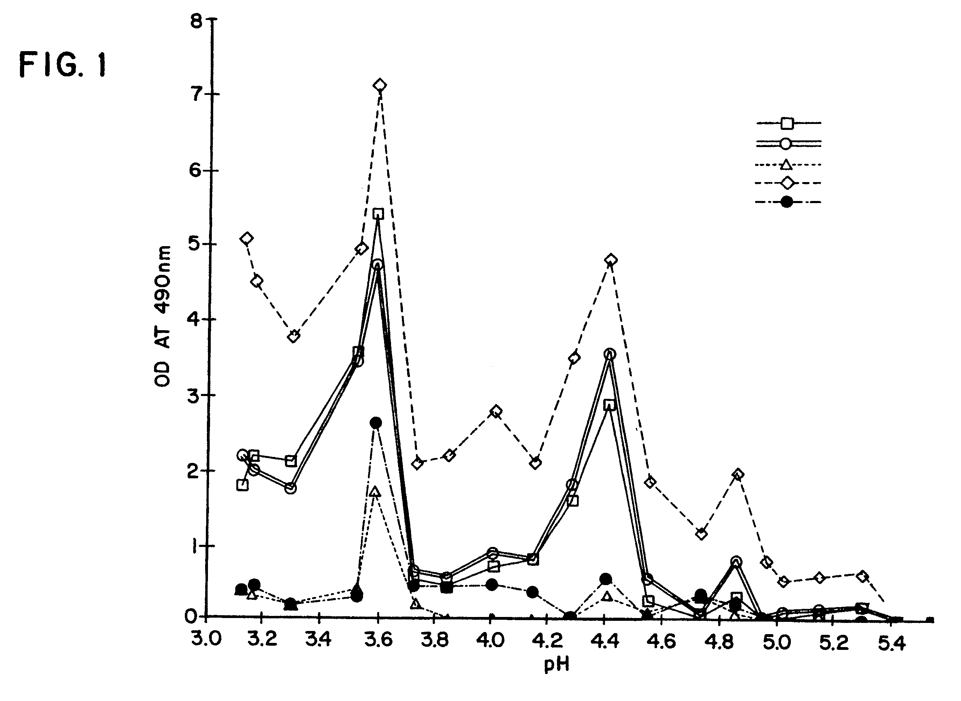

Figure 1 depicts the isoelectric focusing patterns of colon carcinoma cytosol containing the antigen of the present invention examined against a panel of monoclonal antibodies.

Figure 2 shows the biodistribution of $^{111}$In-labeled CCR086 forty-eight hours after injection into T183 tumor-bearing nude mice.

As indicated above, the present invention provides a tumor-associated antigen reactive with monoclonal antibody CCK061, generated by hybridoma cell line ATCC Deposit #HB 8786 (deposited April 19, 1985), and monoclonal antibody CCR086, generated by hybridoma cell line ATCC Deposit #HB 10051 (deposited March 9, 1989).

The physicochemical and immunological properties of the antigen and particularly its reactivity with monoclonal antibodies CCK061 and CCR086, permit its characterization and differentiation from other antigens present in cancerous human cell lines and human tissue, including human tumor tissue. As used herein, cell lines refer to reproducible cells that may be grown in vitro, e.g., in tissue culture, or as xenographs in suitable animal hosts such as nude mice. The unlimited availability of cells from established cell lines distinguish them from the limited availability of cells from tissues, normal and cancerous. The term "tissue specimen" is used interchangeably with the term "tissue", which is a solid sample or other aggregation of cells performing a similar function obtained from surgery, biopsy or autopsy.

Accordingly, the identifying characteristics and properties of the antigen provided are as follows:

a) The antigen is present in human colon carcinoma cell lines. Standard enzyme-linked immunoabsorbent binding assay (ELISA) procedures using monoclonal antibody CCK061 indicate the presence of the antigen in colon carcinoma cell lines but not in other cell lines such as breast carcinoma, prostate carcinoma, bladder carcinoma, lung adenocarcinoma and melanoma cell lines.

b) The antigen is produced in the cytosol of normal colon and colon carcinoma tissues. Reactivity of CCK061 with plasma membranes purified from human colon carcinoma and tissues is shown by conventional ELISA techniques. By comparison, antibody CCK061 exhibits no reactivity with membranes purified from other normal or tumor tissues, including breast carcinoma, lung adenocarcinoma, prostate carcinoma and melanoma.

c) Standard immunohistochemical procedures demonstrate the presence of the antigen in normal colon, colon carcinoma, esophogeal carcinoma and gastric carcinoma as shown by a strong reactivity with antibodies CCK061 and CCR086 in immunoperoxidase staining of such tissues. By comparison, breast carcinoma, lung adenocarcinoma, renal carcinoma, small lung cell carcinoma and melanoma show a weak reactivity or no reactivity with these antibodies.

d) SDS-polyacrylamide gel electrophoresis (SDS-PAGE) and Western immunoblot analysis reveal that the antigen has a molecular weight in the range of about 600 Kd to about 1,000 Kd.

e) Metabolic labeling of colon carcinoma cells with $^{14}$C-glucosamine and immunoprecipitation of the antigen with CCK061 by standard procedures demonstrate the glycoprotein nature of the antigen.

f) Isoelectric focusing of the antigen in solution indicates isoelectric points within the ranges of about 4.3 and about 4.5 and about 4.8 and 5.0, with peaks at about 4.4 and about 4.9.

g) Studies with various mucin preparations suggest that monoclonal antibodies CCR086 and CCK061 have specificities directed to different epitopes of the antigen characterized herein.

Summarizing the foregoing, the tumor-associated antigen is characterized as a substantially pure glycoprotein having a molecular weight within the range of about 600,000 daltons (600 Kd) to about 1,000,000 daltons (1000 Kd) and having isoelectric points within the ranges of about 4.3 to about 4.5 and about 4.8 to about 5.0. More specifically, the antigen is characterized as having isoelectric points at about 4.4. and about 4.9. Furthermore, the tumor-associated glycoprotein is expressed by normal colon and colon carcinoma tissue cells and is present in human colon carcinoma cell lines.

Of particular importance in distinguishing the antigen of the present invention from other antigens, including other tumor-associated antigens, is the specificity of monoclonal antibodies CCK061 and CCR086 for the antigen characterized herein. Additionally, the specificity of CCK061 and CCR086 for the antigen defined by the invention provides a means for the isolation and purification of the antigen from other material of human origin, and ultimately, the characterization of antigenic determinants. Such a purified antigen and determinants thereof are useful in the production of monoclonal and polyclonal antibodies for diagnostic and therapeutic applications using techniques well known in the art. For example, purified antigen may be used to immunize animals to generate murine hybridomas expressing monoclonal antibodies specific for the antigen. In other cases, the antigen may be used to stimulate an immune response in a rabbit, goat or other animal from whose serum polyclonal antibodies may be obtained as described, for example, in Ghose et al., Methods in Enzymology, vol. 93, 326-327 (1983). In addition, the antigen may be used for the characterization of antibodies of interest, e.g., monoclonal antibodies or antibodies present in human tissue or body fluids. In describing the present invention, the term "specificity" is used interchangeably with the terms "specific reactivity" and "immunoreactivity".

In accordance with the present invention, antibodies having specificity for the antigen characterized herein, and methods for their production are provided. Preferably, such antibodies are monoclonal antibodies possessing immunoreactivity with the tumor-associated glycoprotein of the present invention substantially similar to that of the preferred monoclonal antibodies CCK061 and CCR086. Alternatively, antibodies of this aspect of the invention may be polyclonal in origin. The antibodies of the present invention may be useful in the detection, diagnosis and treatment of cancer in humans, particularly colorectal carcinoma. Furthermore, such antibodies may have application in the isolation and purification of the antigen provided by the invention and the characterization of precise determinants.

Monoclonal antibodies as described above may be produced essentially according to the method of Kohler and Milstein, Nature 256, 495-497 (1975) as modified by Gerhard, Monoclonal Antibodies, 370-371, R. Kennett et al. eds. (Plenum Press 1980). In accordance with the present invention, a mouse or other suitable host well known in the art is immunized with the purified antigen of the invention or a soluble fraction of human tumor tissue derived from a colon carcinoma. Following immunization, the spleen cells of the immunized mouse are fused with suitable mouse myeloma cells to obtain a mixture of hybrid cell lines. Resulting cell lines are cultured in media selective for hybrid cell lines. Surviving hybrids producing monoclonal antibodies, preferably CCK061 or CCR086, having specificity for the antigen characterized herein are thereafter cloned and the monoclonal antibodies produced are recovered.

The present invention includes methods for the in vitro detection of cancer in humans, particularly colorectal carcinoma. Characterization of the unique tumor-associated glycoprotein of the invention as set forth herein permits its detection in patient tissue specimens. One method of in vitro detection is by immunohistochemical methods. Immunohistochemical methods for the detection of antigens in patient tissue specimens are well known to those skilled in the art, such as the methods taught in Taylor, Arch. Pathol. Lab. Med. 102, 113 (1978). Briefly, in the context of the present invention, a tissue specimen obtained from a suspected cancer patient is contacted with an antibody, preferably a monoclonal antibody, and more preferably monoclonal antibodies CCK061 and CCR086, having specificity for the tumor-associated antigen of the present invention. The sites at which antibody is bound to antigens is thereafter determined by selective staining of the tissue specimen by standard immunohistochemical procedures. Such procedures include, for example immunoperoxidase staining, avidin-biotin method, and immunofluorescence staining using fluorescein isothiocyanate. As used herein, tissues are solid collections of cells or other aggregation of cells performing a similar function. A qualitative or quantitative determination of the tumor-associated antigen of the present invention in patient specimens by immunohistochemical or immunoassay procedures is of diagnostic utility and may be indicative of or correlate with the progression of a disease state.

Similarly, methods for the in vitro detection of antigenic substances in patient fluid samples by immunoassay procedures are also well known in the art. Fluid samples include serum, plasma, urine, saliva, sweat, ascitic fluid, pleural fluid and other body fluids. For purposes of the present invention, a patient fluid sample may be contacted with at least one antibody, preferably a monoclonal antibody, having specificity for the tumor-associated antigen of the invention and the binding of the antibody to antigen components of the fluid sample determined by methods disclosed herein or known in the art. Qualitative or quantitative determinations of the antigen defined by the invention may be accomplished by competitive or non-competitive immunoassay procedures. Monoclonal antibodies, preferably CCK061 and CCR086, may be used in this aspect of the invention and are preferred. Alternatively, polyclonal antibodies having specificity for the antigen provided by the present invention may be used. Additionally, the immunoassays preferred for use are two-site immunometric assays well-known to those skilled in the art that employ monoclonal antibodies selected to bind to non-interfering determinants of a target antigen. For example, the two-site immunometric assays described in U.S. Patent No. 4,376,110 to David et al., issued March 8, 1983, may be used and that patent is incorporated herein by reference.

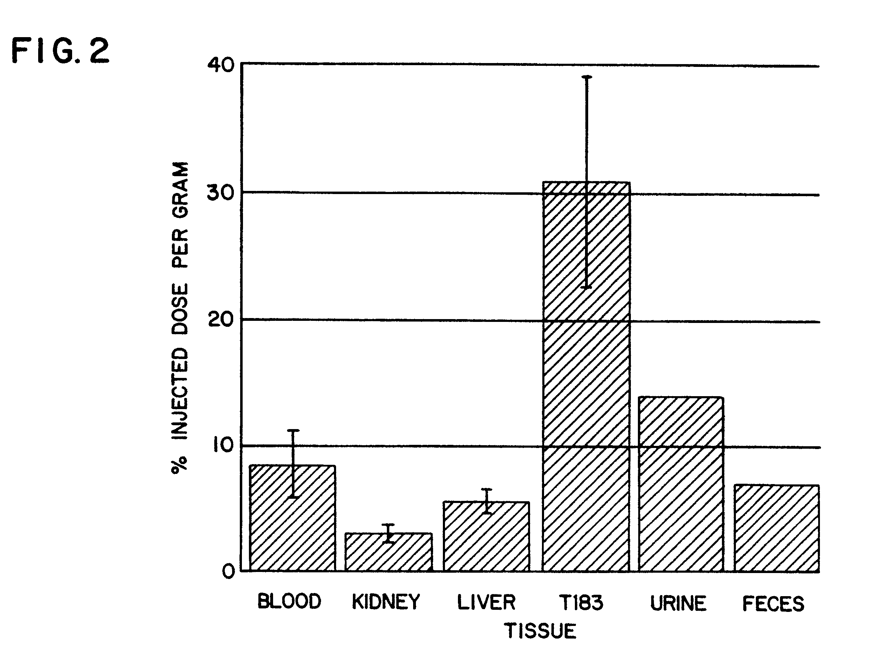

The significant localization of an antibody of the present invention to colorectal carcinoma in tumor-bearing mice, as shown in Fig. 2, suggests the usefulness of the invention in in vivo applications. For example, an aspect of the present invention is the in vivo diagnosis and therapy of cancer in humans, particularly colorectal carcinoma. Methods for tumor localization and detection may be performed, in accordance with the present invention, by administering to a suspected cancer patient a predetermined effective amount of an antibody having specific reactivity with the tumor-associated antigen of the present invention and detecting the sites of localization of the antibody. The sites of localization are determined by standard imaging techniques, preferably planar imaging and/or single photon emission computed tomography (SPECT). The predetermined effective amount of antibody, labeled and unlabeled, for imaging applications is within the range of about 2 to about 200 mg, preferably in the range of about 5 to about 80 mg, and more preferably about 20 to about 40 mg. The antibody, preferably a monoclonal antibody, and more preferably CCK061 or CCR086, is administered to the patient in a pharmaceutically acceptable carrier and labeled with a marker to permit in vitro detection. For imaging, antibodies are preferably labeled with a radioistope such as gamma-emitters, positron-emitters, and x-ray-emitters including, for example, indium-111, technetium-99m, iodine-125, gallium-67, and gallium-68. The antibodies, such as CCK061 and CCR086, are preferably labeled with a gamma-emitting label such as indium-111. Pharmaceutically acceptable carriers for imaging and therapeutic uses are well-known in the art and include aqueous solutions such as bicarbonate buffers, phosphate buffers, Ringer's solution and physiological saline, supplemented with 5% dextrose or human serum albumin, if desired. A further advantage of the present invention is the discovery that certain antibodies of the present invention may detect sites of colorectal carcinoma that are not detected by conventional methods. The course of treatment in such instances may change drastically upon locating tumor sites previously undetected by other methods.

In accordance with methods of the present invention for cancer therapy, a predetermined effective amount of an antibody, preferably a monoclonal antibody having specificity for the tumor-associated antigen characterized by the invention is administered to a diagnosed cancer patient. The predetermined effective amount of antibody for therapeutic applications is in the range of about 1 to about 100 mg, preferably in the range of about 2 to about 40 mg, and more preferably in the range of about 2.5 to about 10 mg. The monoclonal antibody, preferably CCK061 and CCR086 antibodies, is administered to the cancer patient in a pharmaceutically acceptable carrier as described above and conjugated with a suitable therapeutic agent selected for delivery to the tumor site. Therapeutic agents include radioisotopes, drugs, toxins and biological proteins. Radioisotopes include those useful for imaging as well as emitters of alpha and beta particles such as yttrium-90, scandium-47 and iodine-131. Drugs include, in general, alkylating agents, antiproliferative agents, tubulin-binding agents, cytotoxins in general, and the like. Preferred compounds are the nitrogen mustard agents, the vinca alkaloids, the daunomycin family, the mitomycins, the bleomycins, the cytotoxic nucleosides, the pteridine family of drugs, and the sulfonylureas (as described in European Patent Publication No. 222,475, published May 20, 1987). Particularly useful members of those compounds include, for example, doxorubicin, daunorubicin, aminopterin, methotrexate, methopterin, dichloromethotrexate, mitomycin C, porfiromycin, 5-fluorouracil, 6-mercaptopurine, cytosine arabinoside, etoposide, melphalan, vinblastine, vincristine, leurosidine and the like. Toxins suitable as therapeutic agents include the podophyophyllotoxins, ricin, the trichothecenes, the colchicines and pseudomonas endotoxin. Biological proteins having therapeutic value include hormones, the interferons (alpha, beta and gamma), the interleukins and the like. Methods for attaching antibodies to such therapeutic agents for cancer therapy are well-known to those skilled in the art. For example, methods of attaching therapeutic agents to antibodies are disclosed in Blair et al., J. Immunol. Methods 59, 129 (1983), Ghose et al., Methods in Enzymology 93, 280 (1983) and in U.S. Patent No. 4,741,900 to Alvarez et al. issued May 3, 1988 and incorporated herein by reference. Another aspect is the attachment of the present antibodies to a therapeutic agent. Methods of administering the present therapeutic compositions include, intravenous, intraperitoneal, intralymphatic, intrathecal and intraarterial infusion or injection.

In still another aspect, pharmaceutical compositions comprising an antibody having specific reactivity with the antigen characterized herein and a pharmaceutically acceptable carrier as described above are provided. Antibodies used in the preparation of the pharmaceutical compositions of the present invention are preferably monoclonal antibodies, and more preferably the monoclonal antibodies CCK061 and CCR086. The antibodies are also optionally labeled with the therapeutic agents described above.

Additionally, in the context of in vivo cancer diagnosis and therapy, those skilled in the art will appreciate that antibody preparations comprising mixtures of antibodies or fragments thereof having specificity for the described tumor-associated antigen may be used in certain instances to enhance the detection, localization and treatment of tumors.

Further in accordance with the present invention, we have unexpectedly discovered that at least one determinant of the antigen characterized herein is expressed by mucin antigens associated with the genetic disease of cystic fibrosis. More specifically, we have found that monoclonal antibody CCK061 may be useful for the detection of elevated serum levels of mucin antigens indicative of cystic fibrosis. As CCK061 is reactive with a protein epitope of mucin antigens unrelated to the Lewis blood group system, CCK061 is particularly useful for the detection of cystic fibrosis in patients genetically unable to produce detectable quantities of sialosylated Lewis antigens associated with the disease. Accordingly, the present invention suggests a method for the detection of cystic fibrosis by contacting a serum sample with monoclonal antibody CCK061 and determining the binding of CCK061 to sample components by means of an immunoassay. Preferably, a second antibody, capable of binding with mucin antigens associated with cystic fibrosis, is employed in the immunoassay. Particularly preferred for the detection of cystic fibrosis is the use of CCK061 in an immunoassay which is a two-site immunometric assay employing a second antibody which is a monoclonal antibody and selected such that CCK061 and the second antibody bind to non-interfering determinants of serum mucin antigens associated with cystic fibrosis.

The present invention may be better understood by reference to the following non-limiting examples.

## EXAMPLE 1

Production of Monoclonal Antibody CCK061

To produce hybrid cell line ATCC Deposit #HB 8786 generating monoclonal antibody CCK061, female Balb/c mice (Charles River Breeding Laboratories, Wilmington, MA) were immunized intraperitoneally with 200 μg of tumor cytosol obtained from an autopsy specimen of human colon carcinoma (hereinafter designated as SC83-421) five times at 14-day intervals.

Three days after the fifth immunization, spleens were aseptically removed from the mice into AP-MEM media (Flow Laboratories, Inglewood, California). After the spleens were carefully disrupted to release splenoctyes into the media, the clumps of cells were disassociated by pipetting and transferred to a centrifuge tube. Upon standing for about 5 minutes, the cell suspension was removed from the sedimented materials and centrifuged at 1,000 x g for 5 minutes. After washing and a second centrifugation, the resulting splenoctye pellet was resuspended in AP-MEM media.

Cell fusion was carried out according to the procedure of Kohler and Milstein, Nature 256, 495-497 (1975) as modified by Gerhard, Monoclonal Antibodies, R. Kennett et al., 370-371 (Plenum Press 1980). Briefly, $1 \times 10^8$ splenocytes were fused with $2.5 \times 10^7$ P3-X63-Ag8.653 (ATCC #CRL 1580), a mouse myeloma cell line, in 1.0 ml of 35% polyethylene glycol (PEG 1500) in AP-MEM medium. Following the fusion, cells were cultured in HAT medium (hypoxanthine, aminopterin, thymidine) at 37° C in a humidified 5% $CO_2$ incubator.

Antibodies produced by the resulting hybridomas were screened by an enzyme-linked immunoabsorbent binding assay (ELISA) on cytosol preparations of the immunizing colon carcinoma. Antibodies demonstrating a 5-fold or greater reactivity with the tumor cytosol compared with normal lung were selected. As a result of the selection method, monoclonal antibody CCK061 was further characterized and selected for use in the antigen characterization as provided herein.

EXAMPLE 2

Characterization of Monoclonal Antibody CCK061

A. Cell-Line ELISA

Monoclonal antibody CCK061 was screened against a panel of human carcinoma cell lines by ELISA to determine the presence of antigen reactive with the antibody. The cell lines used for screening the reactivity of monoclonal antibody CCK061 are set forth in Table 1 below.

Table 1

| Cell Line | Source |
|---|---|
| SW403 Colon Carcinoma | American Type Culture Collection, Rockville, Maryland (ATCC #CCL230). |
| T84 Colon Carcinoma | Dr. H. Masui, University of California at San Diego Cancer Center, San Diego California. |
| SkMel-28 Melanoma | American Type Culture Collection, Rockville, Maryland (ATCC #HTB72). |
| Calu-3 Adenocarcinoma of Lung | American Type Culture Collection, Rockville, Maryland (ATCC #HTB-55). |
| T47D Breast Carcinoma | Dr. Renato Dulbecco, Salk Institute, La Jolla, California. |
| M14 Melanoma | Dr. Ralph Reisfeld, Scripps Clinic and Research Foundation, La Jolla, California. |
| H907 Bladder Carcinoma | Dr. K.E. Hellstrom, Fred Hutchinson Cancer Research Center, Washington. |
| PC-3 Prostate Carcinoma | Dr. Mark Glassey, University of California at San Diego, La Jolla, California. |
| SW620 Colon Carcinoma | American Type Culture Collection, Rockville, Maryland (ATCC #CCL227) |

Cells obtained from the cell lines listed in Table 1 were maintained in tissue culture flasks until approximately 90% confluent. The flasks containing cells and media were frozen at -20°C until ready for use. The flasks were then brought to room temperature and the cells removed for counting. The cells were adjusted to a concentration of $4 \times 10^6$/ml cells and plated out at $2 \times 10^5$ cells per well onto a Cleveland glass fiber filter plate (VP Scientific) and dried. The cells were then washed 3 times with 0.3% gelatin, 1% bovine serum albumen (BSA) in phosphate buffered saline (PBS: 0.01 M sodium phosphate plus 0.14 M NaCl). Fifty $\mu$l of monoclonal antibody CCK061 (10 $\mu$g/ml) was applied per well and incubated for 1 hour at room temperature. After 5 washes with 0.3% gelatin/PBS, 50 $\mu$l of peroxidase conjugated goat anti-mouse IgG and IgM (Tago Chemicals, Burlingame, CA) diluted 1:4000 was added and incubated for 1 hour. After 6 washes, 200 $\mu$l of 1 mg/ml o-phenylenediamine ("OPD"), .03% $H_2O_2$ in 0.1M citrate phosphate buffer (hereinafter referred to as OPD developing solution) was added to develop color. The plate was incubated in the dark for 30 minutes, followed by the addition of 50 $\mu$l/well 4N $H_2SO_4$. The optical density (O.D.) was read at 490 nm.

As shown by Table 2 below, CCK061 was reactive with colon carcinoma cell lines SW403 and T84, indicating the presence of the antigen characterized herein in human colon carcinoma cell lines. By comparison, CCK061 demonstrated no appreciable reactivity with cell lines such as breast carcinoma, prostate carcinoma, bladder carcinoma, adenocarcinoma of lung and melanoma cell lines.

Table 2

| Monoclonal Antibody CCK061 Reactivity with Human Cell Lines | |
|---|---|
| Cell Line | OD$_{490}$ |
| SW403 Colon Carcinoma | .49 |
| T47D Breast Carcinoma | .03 |
| PC-3 Prostate Carcinoma | .08 |
| H907 Bladder Carcinoma | .01 |
| Calu-3 Adenocarcinoma of Lung | .03 |
| SkMel Melanoma | .03 |
| M14 Melanoma | .05 |
| SW620 Colon Carcinoma | .01 |
| T84 Colon Carcinoma | .40 |

B. Membrane ELISA

The presence of antigen reactive with monoclonal antibody CCK061 in purified membrane fractions of normal human tissues and human tumor tissues was determined by ELISA as measured by absorbance at 490 nm.

Membrane/cytosol fractions were prepared from surgical and autopsy specimens of human tissue collected within ten hours after death and stored at -80° C. A tissue specimen was first homogenized in 4 volumes of 10mM tris-HCl, pH 7.5, 2mM calcium chloride, 2mM phenylmethylsulfonate (homogenization buffer) at 4° C in a dounce homogenizer. All subsequent steps were carried out at 4° C. The homogenate was centrifuged at 1000 x g for 5 minutes to remove nuclei and intact cells. The supernatant was removed and centrifuged at 100,000 x g for one hour. The supernatant from the high speed centrifugation was removed and designated as the cytosol fraction. The membrane-containing pellet was resuspended in one volume of homogenization buffer and layered on a 40%/20% discontinuous sucrose gradient in 10mM tris-HCl, pH 7.2. The gradient was centrifuged at 100,000 x g for 17 hours. Material at the 40%/20% interphase was pipetted off, diluted 5 fold in homogenization buffer and centrifuged for 60 minutes at 100,000 x g. The pellet was resuspended in homogenization buffer, aliquoted and stored at -80° C.

To perform the ELISA, the membrane fractions were dried onto 96-well flat bottom polyvinyl-microtiter plates (Dynatech, Alexandria, VA) at 1 $\mu$g per well. Plates were washed four times with distilled water and then incubated for 30 minutes at room temperature with 20% horse serum in PBS. The buffer was removed and 50 $\mu$l of CCK061 antibody (10 $\mu$g/ml) applied for one hour. Plates were washed 6 times with tap water prior to the addition of 50 $\mu$l/well of peroxidase-conjugated goat anti-mouse IgG and IgM (1:1000 dilution). The plates were then incubated for one hour followed by 5 washes with distilled water. Color was developed after the addition of 100 $\mu$l of the OPD developing solution. The plates were incubated in the dark for 30 minutes, followed by the addition of 50 $\mu$l per well of 4N H$_2$SO$_4$ to quench the reaction. Reactivity was measured at an O.D. of 490 nm.

As shown by Table 3, monoclonal antibody CCK061 was reactive with membrane fractions of normal colon and colon carcinoma but unreactive with membranes of other normal tissues and carcinomas, including breast carcinoma, adenocarcinoma of lung, melanoma, and prostate carcinoma. Accordingly, the antigen characterized by monoclonal antibody CCK061 was demonstrated to be present only in membranes of colon carcinoma and normal colon.

8

Table 3

| Monoclonal Antibody CCK061 Reactivity with Membrane Preparations of Human Tissue | | | |
|---|---|---|---|
| Tumor Membranes | OD$_{490}$ | Normal Membranes | OD$_{490}$ |
| Colon Carcinom | 2.05 | Colon | 2.81 |
| Colon Carcinoma | .78 | Liver | .01 |
| Breast Carcinoma | .00 | Lung | .00 |
| Breast Carcinoma | .03 | Pancreas | .00 |
| Lung Adenocarcinoma | .03 | Spleen | .00 |
| Lung Adenocarcinoma | .01 | Breast | .00 |
| Squamous Lung Carcinoma | .01 | Bladder | .00 |
| Small Cell Lung Carcinoma | .00 | Brain | .00 |
| Small Cell Lung Carcinoma | .00 | | |
| Melanoma | .01 | | |
| Prostate Carcinoma | .01 | | |
| Prostate Carcinoma | .00 | | |

C. Immunohistochemical Determination of CCK061

The presence of antigen reactive with CCK061 in normal and tumor tissue was determined by immunoperoxidase staining of human tissue.

An indirect immunoperoxidase assay, essentially as described by Taylor, Arch. Pathol. Lab. Med. 102, 113 (1978), was used to stain the sections. Frozen tissue blocks, obtained from surgical and autopsy specimens collected within ten hours after death and stored at -80°C, were sliced into 4-6 micron sections on a microtome/cryostat and mounted on gelatin-coated glass slides. The sections were briefly air-dried, dipped in acetone, and then rehydrated by incubating in PBS for 5 minutes. Following overlay with supernatant containing CCK061 antibody at 10 µg/ml, the sections were incubated in a humid chamber for 1 hour. The sections were then washed with PBS to remove unbound antibody and immersed in PBS for 5 minutes. After overlaying the sections with a 1:50 dilution of peroxidase-conjugated goat anti-mouse IgG and IgM antibody (Tago Chemicals, Burlingame, CA), they were incubated for 30 minutes in a humid chamber, followed by washings with PBS to remove unbound secondary antibody. Color was developed by the addition of 1 mg/ml of diaminobenzidine and 0.03% $H_2O_2$ and thereafter counter-stained with hematoxylin eosin.

The results, summarized in Table 4 below, indicate that monoclonal antibody CCK061 exhibits strong reactivity with normal colon mucosa, as measured by the intensity of staining of such tissue. By comparison, CCK061 antibody demonstrated weak or no reactivity with other normal tissues.

Table 4

| Immunohistological Reactivity of CCK061 on Normal Tissue | |
|---|---|
| Tissue | No. Positive/Total Examined |
| Strong Reactivity | |
| Colon (mucosa only) | 4/4 |
| Weak Reactivity | |
| Breast | 1/4 |
| Lung | 3/5 |
| Cervix (variable)[1] | 2/2 |
| Esophagus (variable)[1] | 2/2 |
| Prostate | 1/4 |
| Ovary (variable)[1] | 1/2 |
| Thyroid | 1/2 |
| Pancreas | 1/2 |
| No Reactivity | |
| Liver | 0/2 |
| Stomach | 0/2 |
| Kidney | 0/2 |
| Bladder | 0/1 |
| Uterus | 0/1 |
| Small Intestine | 0/2 |
| Testis | 0/2 |

[1]Variable = non-homogeneous staining

As shown in Table 5 below, monoclonal antibody CCK061 is strongly reactive with colon carcinoma, esophogeal carcinoma and gastric carcinoma, and weakly or not reactive with other carcinomas. In particular, a weak reactivity was shown with carcinomas such as breast carcinoma and lung adenocarcinoma.

Table 5

| Immunohistological Reactivity of CCK061 on Tumor Tissue | |
| --- | --- |
| Tissue | No. Positive/Total Examined |
| Strong Reactivity | |
| Colon Carcinoma | 15/18 |
| Esophogeal Carcinoma | 2/2 |
| Gastric Carcinoma | 2/2 |
| Weak Reactivity | |
| Breast Carcinoma (luminal ducts) | 3/7 |
| Adenocarcinoma of Lung | 1/4 |
| Squamous Carcinoma | 2/4 |
| Pancreatic Carcinoma | 2/4 |
| No Reactivity | |
| Small Cell Lung | 0/4 |
| Prostate Carcinoma | 0/4 |
| Hepatoma | 0/2 |
| Melanoma | 0/1 |
| Brain Carcinoma | 0/2 |
| Renal Carcinoma | 0/2 |

D. Isotype Determination of CCK061

Goat anti-mouse $IgG_1$, $IgG_2$, $IgG_{2B}$, $IgG_3$ and IgM (Tago, Burlingame, California) were first diluted 1:3000 in a 10mM sodium phosphate buffer solution (pH 7.2) from stock concentrations of 1 mg/ml protein. The test wells of a 96-well polyvinyl microtiter plate were first coated with the diluted goat anti-mouse Ig at 50 µl per well and incubated overnight at 37°C. The plate was then washed with PBS-0.1% Tween followed by a second wash with distilled water. To each test well, 200 µl of a blocking solution was added to each well and the covered plate stored at 4°C. The blocking solution was prepared by slowly adding 20 gm BSA (Sigma), 2 ml Tween-20 (Sigma) and 20 ml 10% sodium azide to 2 liters of PBS with constant stirring.

Within five days, the plate was washed with PBS-0.1% Tween followed by a second wash with distilled water at room temperature. Next, 40 µl of antibody supernatant, positive IgG and IgM controls and HAT media (used as a negative control) were added to the test wells, covered, and incubated at 37°C for one hour. After washing the plate three times with PBS-0.1% Tween and once with distilled water, 100 µl of freshly made OPD developing solution was added to each well. The plate was immediately covered with foil and incubated at room temperature for 15 minutes with shaking. To stop the reaction, 50 µl of 4N $H_2SO_4$ was added to each well and the plate was read at 490 nm on an ELISA reader.

Following the procedure described above, the isotype of monoclonal antibody CCK061 was determined to be of the murine IgM class.

EXAMPLE 3

Production of Monoclonal Antibody CCR086

11

Monoclonal antibody CCR086 may be recovered from a hybridoma produced by the method described in Example 1. Alternatively, monoclonal antibody CCR806 generated by hybridoma cell line ATCC #HB 10051 was produced by the method of Kohler and Milstein, Nature 256, 495-497 (1975) as modified by Gerhardt, Monoclonal Antibodies 370-371, R. Kennett et al. (Plenum Press 1980) and further modified by co-priming Balb/c mice with monoclonal antibodies CCO135 and CCP137 specific for human colon carcinoma antigens found in normal tissue. On weeks 1 and 4 of the immunization protocol, Balb/c mice were injected intravenously with 100 μg of 5 mg/ml CCO135 ascites and 100 μg of 5 mg/ml CCP137 as ascites. The hybridomas generating monoclonal antibodies CCO135 and CCP137 were produced at Hybritech Incorporated, assignee of the present invention. On weeks 3 and 7, the same Balb/c mice were injected intraperitoneally with 50 μg of membranes prepared from a human colon carcinoma specimen (SC83-421) obtained at autopsy within 10 hours after death and stored at -80° C.

Three days following the final immunization, spleens were aseptically removed and the splenocytes separated to form a cell suspension. The splenocytes were then fused with P3-X63-Ag.8.653 myeloma cells according to the fusion procedure set forth in Example 1.

Antibodies produced by the resulting hybridomas were screened by ELISA on cytosol preparations of the SC83-421 human colon carcinoma specimens. Antibody CCR086 demonstrated greater reactivity with the tumor cytosol compared to normal liver cytosol. Consequently, CCR086 was selected for further characterization for use in the antigen characterization as provided herein. Based on these results, CCR086 was further selected for in vivo studies to determine the effectiveness of using the antibodies of the present invention to detect and treat human colorectal carcinoma.


EXAMPLE 4


Characterization of Monoclonal Antibody CCR086


A. Isotype Determination

Using the procedure described in Example 2D, the isotype of monoclonal antibody CCR086 was determined to be of the murine IgG₁ class.


B. Immunohistochemical Determination

The immunohistochemical characterization of the CCR086 antibody was determined by its reactivity to a panel of normal and tumor tissues. Tissue specimens were obtained from surgery and autopsy collected within ten hours of death and stored at -80° C. The frozen specimens were sliced into 4-6 micron sections and mounted onto gelatin-coated glass slides. The sections were then air-dried, fixed in acetone for 5 minutes, and treated with 5% normal goat serum. The reactivity of the CCR086 antibody with the panel of normal and tumor tissues was evaluated by the indirect immunoperoxidase assay described in Example 2C. The results of the evaluation are summarized below in Table 6 for human tumor tissue and Table 7 for human normal tissue. As shown in Tables 6 and 7, monoclonal antibody CCR086 is reactive with a subset of normal colon and prostate tissue and a subset of their derived tumors. By comparison, monoclonal antibody CCR086 demonstrated no reactivity with all other normal and tumor tissues examined.

TABLE 6

| Monoclonal Antibody CCR086 Reactivity with Human Tumor Tissue | |
| --- | --- |
| Tissue | No. Positive/Total Examined |
| Colon carcinoma, mucinous | 11/16 |
| Colon carcinoma, non-mucinous | 5/9 |
| Prostate carcinoma | 2/7 |
| Breast carcinoma | 0/8 |
| Gastric carcinoma | 0/4 |
| Lung Adenocarcinoma | 0/8 |
| Lung Broncheo-Alvealor carcinoma | 0/2 |
| Lung Epidermoid carcinoma | 0/2 |
| Lung Large Cell carcinoma | 0/2 |
| Lung Small Cell carcinoma | 0/2 |
| Lymphoma | 0/2 |
| Melanoma | 0/4 |
| Pancreatic carcinoma | 0/3 |
| Rectal carcinoma | 0/2 |
| Renal carcinoma | 0/4 |
| Sarcoma | 0/2 |
| Testicular carcinoma | 0/4 |
| Thyroid carcinoma | 0/2 |

EP 0 397 419 A2

TABLE 7

| Monoclonal Antibody CCR086 Reactivity with of Human Normal Tissue | |
| --- | --- |
| Tissue | No. Positive/Total Examined |
| Colon | 8/11 |
| Prostate | 3/8 |
| Adrenal | 0/2 |
| Bladder | 0/2 |
| Brain | 0/2 |
| Breast | 0/8 |
| Cervix | 0/2 |
| Diaphragm | 0/1 |
| Duodenum | 0/3 |
| Esophagus | 0/2 |
| Heart | 0/2 |
| Ileum | 0/1 |
| Jejunum | 0/3 |
| Kidney | 0/6 |
| Liver | 0/6 |
| Lung | 0/10 |
| Lymph Node | 0/2 |
| Ovary | 0/2 |
| Pancreas | 0/2 |
| Peripheral Nerve | 0/11 |
| Placenta | 0/2 |
| Salivary Gland | 0/1 |
| Skin | 0/1 |
| Spleen | 0/2 |
| Spinal Cord | 0/1 |
| Stomach | 0/2 |
| Testes | 0/2 |
| Thymus | 0/1 |
| Thyroid | 0/1 |
| Tonsil | 0/2 |
| Urethra | 0/1 |
| Uterus | 0/2 |
| Vagina | 0/1 |

A similar staining pattern was observed in immunoperoxidase assays with non-human primate cynomolgous tissues. Table 8 below provides a comparison of reactivity between corresponding normal human and cynomolgous tissues with monoclonal antibody CCR086.

14

TABLE 8

| Comparison of Human/Primate Normal Tissue Reactivity with Monoclonal Antibody CCR086 | | |
|---|---|---|
| Tissue (Benign) | Human[1] | Cynomolgous[1] |
| Colon | 2/2 | 2/2 |
| Duodenum | 0/2 | 0/2 |
| Jejunum | 0/2 | 0/2 |
| Kidney | 0/2 | 0/2 |
| Liver | 0/2 | 0/2 |
| Lung | 0/2 | 0/2 |
| Ovary | 0/1 | 0/1 |
| Pancreas | 0/2 | 0/2 |
| Spleen | 0/2 | 0/2 |
| Testis | 0/1 | 0/1 |
| [1]No. Positive/Total Examined | | |

In pre-clinical primate toxicology studies with tissues from macaca fascicularis (cynomolgous) monkeys, monoclonal antibody CCR086 demonstrated no clinically detectable toxicity at doses up to 11.5 times the maximum weight adjusted human imaging dose of 20 mg/70 kg.

C. Cytosol ELISA

Monoclonal antibodies CCR086, CCK061 and ZBC062 were tested against bovine submaxillary mucin (BSM), carcinoembryonic antigen (CEA) extracted from the T84 cell line, and colon carcinoma cytosol from tissue specimen (SC83-421). Five µg/well of BSM and CEA and 1 µg/well of colon cytosol were first dried onto test wells. The ELISA procedure as described in Example 2A was generally followed. The dried test wells were incubated with 10µg/ml CCR086, CCK061 or ZBC062. Goat anti-mouse IgG-Biotin (Zymed) diluted 1/1000 was used as the secondary antibody. The results of the ELISA are summarized in Table 9 below and suggest that CCR086 may be useful as a non-CEA tumor marker for targeting colon cancer.

This suggestion was further confirmed in another study in which CCR086 failed to react with CEA in a Western blot analysis. The details of the Western blot analysis are provided below in Example 8.

TABLE 9

| Reactivity with BSM, Colon Carcinoma Cytosol and CEA | | | |
|---|---|---|---|
| | BSM | SC83-421 | CEA |
| CCR086 | - | + | - |
| CCK061 | - | + | + |
| ZBC062 | + | - | - |
| + = positive reactivity | | | |
| - = negative reactivity | | | |

EXAMPLE 5

15

Purification of Monoclonal Antibody CCR086

Monoclonal antibody CCR086 was purified from 204 ml of pooled ascites fluid obtained from pristane-primed Balb/c mice injected intraperitoneally with 1 x 10^7 CCR086 hybridoma cells (ATCC #HB 10051). Ascites preparation and harvesting methods are described in Galfre and Milstein, Methods in Enzymology, Vol. 73B, 43-45, Langone & Van Vunakis, eds. (Academic Press 1981).

The pooled ascites fluid was centrifuged at 4°C for 20 minutes at 15,000 x g. The antibodies were precipitated from the supernatant fraction by adding 478 ml of a 25% sodium sulphate solution with continuous slow stirring at room temperature. The resulting mixture was allowed to stand for 1.5 hours at room temperature and thereafter centrifuged at room temperature for about 20 minutes at 15,0000 x g. Following the removal of the supernatant fraction, which was discarded, 120 ml of 18% sodium sulfate was then added to the precipitated material to form a homogenous solution and centrifuged at room temperature for 20 minutes at 15,000 x g. The precipitate was dissolved in 62 ml of 50mM sodium phosphate (pH 8.2) and dialyzed at 4°C against 50mM sodium phosphate buffer solution (pH 8.2) for 24 hours with one change of buffer after 16 hours. The dialyzed antibody solution was then diluted with sterile water to a total volume of 324 ml and centrifuged at 15,000 x g for 20 minutes at 4°C. The supernatant fraction containing CCR086 antibody was carefully removed for further purification.

A DEAE Sephacel (Pharmacia) column equilibrated with 0.01 M sodium phosphate (pH 8.2, conductivity 1.8) was used to purify the precipitated antibody. After loading the antibody solution onto the DEAE column with 0.01 M sodium phosphate (pH 8.2, conductivity 1.8), the column was first washed with 0.025 M sodium phosphate buffer solution (pH 8.2, conductivity 3.6), and then eluted with 0.05 M sodium phosphate (pH 8.2, conductivity 6.4). Fractions having an absorbance greater than 1.0 at 280 nm were collected, pooled and stored at 4°C.

EXAMPLE 6

Preparation of $^{111}$In-Labeled CCR086

Radiolabeling CCR086 with Indium-111 was accomplished through the chelating agent diethylenetriamine-pentaacetic acid (DTPA). First, 3.6 ml of 10mM DTPA (pH 9.5) was added to 8.8 ml of 20.45 mg/ml purified CCR086 antibody. The pH of the antibody-DTPA solution was adjusted to about 9.5 using 1.0 M Na$_2$CO$_3$ (pH 12). Next, the antibody concentration was adjusted to 5 mg/ml by adding 23.6 ml of 95mM NaHCO$_3$ with gentle stirring for 15 minutes at room temperature. While the antibody solution was stirring, an $^{111}$In(III)isothiocyanate solution (ITC solution) was prepared by adding 0.58 ml of 0.6mM sodium phosphate (pH 8.2) to 0.58 ml of 37mM $^{111}$In(III)isothiocyanate. Immediately after preparing the ITC solution, 1.16 ml of the solution was added to the antibody-DTPA solution followed by continuous stirring at room temperature for 2.5 hours. The reaction was thereafter quenched by cooling to 0°C.

After loading the labeled antibody conjugate mixture onto a P-6DG Sephadex column, the column was eluted with 0.13 ammonium citrate (pH 6.0) at a 70 ml/hr flow rate. Using a UV monitor, all fractions of the eluant having an absorbance (A$_{280}$) of greater than 0.1 were collected and tested for $^{111}$In incorporation. Fractions having the highest incorporation were pooled, resulting in a pool volume of 33.2 ml to which 0.63 ml of 25% normal serum albumin added to a pool volume of 33.2 ml. The resulting antibody solution was adjusted to a final concentration of 1 mg/ml with 109 ml of 0.13 ammonium citrate (pH 6.0), sterile filtered and stored at 4°C.

Tests to compare the reactivity of unconjugated and conjugated CCR086 antibody were performed to determine the effect of conjugation on the reactivity of the antibody. Purified and conjugated CCR086 antibody were tested in ELISA against 1 μg/well cytosol extracted from colon carcinoma tissue specimen (SC83-421) prepared according to the method described in Example 2B . The results, as shown in Table 10, indicate that the conjugated form of CCR086 exhibits no significant loss of reactivity compared with the unconjugated form.

16

TABLE 10

| Reactivity of Purified and Conjugated CCR086 Antibody | | | | | | |
|---|---|---|---|---|---|---|
| | Dilutions of CCR086 ($\mu$g/well) | | | | | |
| Sample | 10 | 2.5 | 0.625 | 0.156 | 0.039 | 0.0098 |
| purified | 4.51 | 5.32 | 4.30 | 1.85 | 0.54 | 0.15 |
| conjugated | 4.24 | 4.86 | 4.24 | 1.90 | 0.63 | 0.18 |

EXAMPLE 7

Antigen Characterization: Capture RIA

To determine whether monoclonal antibodies CCK061 and CCR086 recognize the same antigen, the antibodies were tested in a capture radioimmunoassay (RIA). As the initial step, 1 $\mu$g of a first antibody (unlabeled CCR086, CCK061, ZBC062, and GDJ352) was plated onto a solid support in each test well of a 96-well polyvinyl microtiter plate and incubated for 1 hour at 4°C. Following a wash with deionized water, 1 or 5 $\mu$g/well of either colon carcinoma cytosol extracted from colon carcinoma tissue specimen (SC83-421) or CEA prepared from T84 extracts were added to the test wells and incubated for 3 hours at room temperature with rotation. After adding 500,000 cpm/well of $^{111}$In-labeled CCR086 or GDJ352, the plate was covered and incubated overnight at room temperature. Prior to cutting the wells for counting on a gamma-counter, the plate was washed several times with water. The results of the capture RIA study, as summarized in Table 11 below, indicate that monoclonal antibodies CCK061 and CCR086 recognize the same antigen. The ability of both CCK061 and CCR086 to bind to the same antigen suggests that the monoclonal antibodies are specific for different epitopes on the same antigen.

TABLE 11

| Capture RIA with $^{111}$In-Labeled CCR086 | | | | |
|---|---|---|---|---|
| | Cytosol | | CEA | |
| Primary Antibody | (1$\mu$g) | (5$\mu$g) | (1$\mu$g) | (5$\mu$g) |
| CCK061 | 18,311 | 27,416 | 94 | 73 |
| CCR086 | 40,321 | 60,692 | 273 | 293 |
| ZBC062 | 428 | 831 | 69 | 90 |
| GDJ352 | 216 | 407 | 78 | 73 |

Although monoclonal antibody GDJ352, an anti-gardnerella hybridoma produced at Hybritech Incorporated, was used as a negative control in this and other experiments described herein, any antibody directed against a non-mammalian antigen may be used, including for example anti-chlamydia or anti-bacterial monoclonal antibodies such as ATCC #9723 and ATCC #12792.

EXAMPLE 8

EP 0 397 419 A2

Antigen Characterization: Molecular Weight Determination

The molecular weight of the novel antigen of the present invention was determined by its reactivity with monoclonal antibodies CCK061 and CCR086 using sodium dodecyl sulfate-polyacrylamide gel electrophoresis (SDS-PAGE) and Western Immunoblot analysis. Cytosol prepared from the colon carcinoma cell line T183 nude mice xenographs was the source of the target antigen. The T183 cell line was obtained from Dr. Nathan Kaplan, University of San Diego, and is described in Zirvi, Cancer Research 42, 3793-3797 (1982). For comparison purposes, cytosol from two human colon carcinoma tissue specimens (designated as SC83-431 and A00343-01), obtained from surgery or autopsy within 10 hours of death, and bovine submaxillary mucin (Sigma) were also tested against a panel of antibodies, specifically CCR086, CCK061, GDJ352, ZBC062 and ZCE063.

To reduce the antigen, the cytosol and mucin preparations (5 $\mu$g/sample) were dissolved in gel sample buffer (62.5mM Tris-HCl pH 6.8, 3% SDS, 10% glycerol, 0.001% bromophenol blue, 5% mercaptoethanol), boiled for 5 minutes and separated by discontinuous SDS-PAGE as described by V.K. Laemmli et al., Nature 227, 680 (1970) on a 3-10% linear gradient gel with a 3% stacking gel. The separated proteins were electrophoretically transferred to nitrocellulose sheets (Schleicher and Schuell) at 250 milliamps for six hours according to the method of H. Towbin et al., PNAS 76, 4350 (1979). The nitrocellulose replicas were first incubated with 3% bovine serum albumin (BSA) in PBS for 30 minutes and then incubated overnight with 10 $\mu$g/ml antibody. The nitrocellulose strips were washed with PBS-0.1% Tween for 30 minutes with 5 changes of buffer and subsequently incubated with 500,000 cpm/ml $^{125}$I-labeled sheep anti-mouse immunoglobulin [Tago] for 3 hours. After washing with PBS-0.1% Tween, the nitrocellulose strips were air-dried and exposed on x-ray film (Kodak XAR-5) for 4-72 hours with intensifying screen at -70° C.

Western blot analysis of the T183 cytosol indicates that the antigen exists as multiple species of high molecular weight ranging from about 600 Kd to about 1,000 Kd. The Western blot patterns for monoclonal antibodies CCK061 and CCR086 reactive with T183 Cytosol are identical, thus providing further support that the antibodies recognize the same antigen. A major band appears at a range of about 650 - 800 Kd with a higher molecular weight smear extending above the major band. In another Western blot analysis, the patterns for CCK061 and CCR086 reactive with purified CEA differed. Specifically, CCK061 reacted with purified CEA migrating at 180 Kd, while CCR086 failed to react with CEA. The molecular weight markers (polymers with phosphorylase b) used in the determination were obtained from Sigma (St. Louis, MD) and range from 97.4 Kd to 584.4 Kd.

In addition to determining the molecular weight of the novel antigen, Western immunoblots show that ZCE063 also reacts with T183 cytosol and exhibits a similar banding pattern as the monoclonal antibodies CCR086 and CCK061. ZCE063, however, failed to react with the SC83-421 tissue cytosol. By comparison, CCR086 and CCK061 reacted with the SC83-421 tissue specimen, but failed to react with A00343-01 or bovine mucin. Finally, ZBC062 reacted with bovine mucin, but failed to react with any of the cytosol preparations. These results are summarized in Table 12.

TABLE 12

| Western Blotting of Colorectal Carcinoma Antibodies | | | | |
|---|---|---|---|---|
| | T183 | SC83-421 | A00343-01 | Bovine Mucin |
| CCK061 | + | + | - | - |
| CCR086 | + | + | - | - |
| ZCE063 | + | - | - | - |
| ZBC062 | - | - | - | + |
| GDJ352 | - | - | - | - |
| + = reactivity - = no reactivity | | | | |

18

## EXAMPLE 9

Antigen Characterization: Glycoprotein Determination

The antigen characterized herein was determined to be a glycoprotein by immunoprecipitation of $^{14}$C-glucosamine labeled SW403 colon carcinoma cells.

SW403 cells were grown in media supplemented with 8% horse serum, and 2% fetal calf serum, to confluency in 75 cm$^2$ tissue culture flasks. After adding 50 $\mu$Ci/ml of $^{14}$C-glucosamine (New England Nuclear, Boston, MA) to the media, the cells were then incubated for 18 hours at 37°C in a humidified 5% $CO_2$ incubator. The cells were thereafter washed three times with PBS, scraped from the flask, and added to 10 ml PBS. The cell pellet was washed and centrifuged an additional 3 times with PBS before being resuspended in 1.0 ml lysis buffer (0.02M Tris-HCl pH 8.0, 1mM EDTA, 0.5% NP-40, 0.5% deoxycholate, 0.5mM phenylmethylsulfonate). After incubation on a rotator at 4°C for 1.5 hours, the lysate was centrifuged in a microfuge (16,000 x g) for 1 minute and the supernatant dialyzed against TBS (0.02M Tris-HCl pH 8.0, 0.15M NaCl, 0.1% sodium azide). The $^{14}$C-labeled lysate was incubated overnight with the CCK061 antibody at 4°C on a rotator. Sepharose-bound sheep anti-mouse immunoglobulin was added to the mixture, incubated 3 hours at room temperature, and thereafter washed 4 times with 0.05M tris, 0.15M NaCl, 0.5% NP-40, 0.05% deoxycholate, 0.1% NaN$_3$, 1 mg/ml BSA pH 8.1 and another 4 times with the same buffer minus the BSA. Samples were separated by SDS-PAGE and the results show that CCK061 precipitates a $^{14}$C-labeled band migrating at approximately 800 Kd.

## Example 11

## Isoelectric Focusing

Isoelectric focusing of the antigen characterized by CCK061 and CCR086 was determined using an isoelectric focusing column as described below.

Twenty mg of T183 colon carcinoma cytosol prepared as described in Example 2B were focused in a water-jacketed 110 ml isoelectric focusing column in a 0-47% sucrose gradient containing ampholytes of the pH range 3.5-10. The column was pre-focused at 600 volts for 12 hours at 4°C prior to injecting each sample into the middle of the gradient followed by focusing for an additional 30 hours at 4°C. One ml fractions were collected from the column and the pH was immediately measured. Collected fractions were dialyzed overnight against 10mM sodium phosphate pH 7.0. Aliquots were examined for reactivity with the CCK061 and CCR086 antibodies in an ELISA assay against cytosol preparations of the T183 colon carcinoma cells.

Isoelectric focusing of the antigen by this technique revealed isoelectric points within the ranges of about 4.3 to about 4.5 and about 4.8 to about 5.0, with peaks of about 4.4 and about 4.9. The major immunoreactive form of the antigen in this tumor has an isoelectric point in the range of about 4.3 to about 4.5.

The peak of activity of all antibodies, including GDJ352 at pH 3.7 probably represents denaturation of antigen in the acidic region of the column.

## Example 12

Antigen Characterization: Epitope Analysis

A. Mucin Studies

Three sources of mucin, human salivary mucin, Lewis a (Le$^a$) mucin, and bovine mucin (Sigma), were examined in an ELISA format for reactivity with a panel of anti-colon carcinoma antibodies. The panel of antibodies included CCR086, CCK061, ZBC062 and ZCE063. The human salivary mucin and Le$^a$ mucin were obtained from Dr. Ginsberg of The National Cancer Institute and may be prepared according to methods well known in the art, including those described in Woodward et al., Biochemistry 21, 694-701 (1982).

To test the reactivity of the mucins against the panel of antibodies, each mucin preparation was first plated out at 1 μg/well onto 96-well microtiter plates and dried overnight at 37°C. After 4 washes with distilled water, the wells were incubated with PBS-20% horse serum for 30 minutes at room temperature followed by a second incubation with antibody at about 1 μg/well for 1 hour. Plates were washed 6 times before adding peroxidase-conjugated goat anti-mouse IgG and IgM (Zymed 1:1000 dilution) at 50 μl/well. Color was developed by addition of OPD developing solution as described in Example 2A and incubated in the dark with shaking for 30 minutes. The reaction was quenched by the addition of 50 μl/well 4N H$_2$SO$_4$ and the O.D. measured at 490 nm. The results of the mucin studies are set forth below in Table 13.

TABLE 13

| Mucin Studies | | | |
|---|---|---|---|
| Antibody | Human Salivary Mucin | Lewis a Mucin | Bovine Mucin |
| CCR086 | - | - | - |
| CCK061 | + + + | + + + | - |
| ZCE063 | - | + | - |
| ZBC062 | ND | ND | + + + |
| + + +: strong reactivity | | | |
| +: weak reactivity | | | |
| -: no reactivity | | | |
| ND: no data | | | |

As shown in Table 13, the monoclonal antibody CCK061 reacted strongly with the two human mucins, while CCR086 exhibited no reactivity with any of the mucin preparations. The results of this study provide additional evidence that monoclonal antibodies CCK061 and CCR086 recognize different epitopes.

B. Destructive Treatment Studies

To determine the nature of the epitope recognized by monoclonal antibody CCK061, 20 μg of cytosol fractions prepared from human tumor specimen SC83-421 as described in Example 2B were subjected to the following treatments prior to SDS-PAGE and Western immunoblot analysis:

a) Sodium Periodate: incubated in 50mM sodium periodate for one hour at 4°C.

b) Neuraminidase: adjusted to pH 5-6 with acetic acid and incubated for 1 hour at 37°C with 10mU of Clostridium perfringens neuraminidase.

c) Proteinase K: incubated in 2.0 mg/ml proteinase K for 1 hour at 37°C.

d) Mild Alkali treatment: incubated overnight in 0.1N NaOH and then neutralized with 0.1N HCl. All reactions were stopped by adding gel sample buffer prior to SDS-PAGE and Western immunoblot analysis as described in Example 8.

Treatments (a) and (b) are designed to destroy carbohydrate sites, while treatments (c) and (d) are designed to destroy the conformation of proteins. Reactivity of the antigen with CCK061 was shown to be destructible by treatment with proteinase K, thus demonstrating the protein nature of the epitope recognized by CCK061. No loss of reactivity resulted from treatment by the other methods described above.

In further experiments, 20 μg of cytosol fractions from SC83-421 prepared as described in Example 2B were subjected to the following destructive treatments and tested in an ELISA format to determine the nature of the epitopes recognized by monoclonal antibodies CCK061 and CCR086:

a) Methanol: incubated in 95% methanol for 1 hour at 4° C.

b) Neuraminidase: incubated for 1 hour at 37° C with 100 units/ml of <u>C. perfringens</u> neuraminidase.

c) Periodate: incubated in 1mM sodium periodate for 30 minutes at room temperature, and reduced with 10mM sodium borohydride.

d) Heat: cytosol was subjected to 100° C for 20 minutes.

e) Reduction/Alkylation: incubated in 6M guanidine-HCl, 10mM DTT for 4 hours at 45° C, followed by incubation in 10mM iodoacetic acid for 30 minutes at 23° C.

f) Urea: incubated in 8M urea for 18 hours at 45° C.

The results of the destructive treatment studies for CCK061 and CCR086 are set forth in Table 14 below. The treated antigens were plated out at 1 μg/well and dried overnight at 37° for testing in the ELISA format as described in Example 2A. For CCR086, the treatments designed to identify carbohydrate epitopes (neuraminidase and periodate) had no significant effect. Treatment with methanol used to identify lipid epitopes likewise had no significant effect. The guanidine and urea treatments also failed to alter the reactivity of CCR086 with the antigen suggesting that the epitope is not a conformational protein epitope unless these denaturation conditions were not effective in destroying the three dimensional structure. The effect of heat on the epitope was inconclusive. Nonetheless, the combined results suggest that CCR086 reacts with a protein epitope.

TABLE 14

| Monoclonal Antibodies CCK061 & CCR086 Destructive Treatment of SC83-421 Cytosol | | | |
|---|---|---|---|
| Treatment | % activity Remaining (average) | Range of Controls | Conclusions |
| Control | 100 | | |
| CCK061 | | | |
| Methanol | 108 | (+) 0-20% (-) 70-100% | non-lipid epitope |
| Neuraminidase | 102 | (+) 0-40% (-) 88-100% | non-sialic acid epitope |
| Periodate | 104 | (+) 0-30% (-) >75% | non-carbohydrate epitope |
| Heat | 64 | (+) 0-6% (-) ≧50% | non-conformatio-nal protein epitope |
| 6M Guanidine | 93 | (+) ≦20% (-) ≧70% | non-conformatio-nal protein epitope |
| 8M Urea | 108 | (+) ≦30% (-) ≧75% | non-conformatio-nal protein epitope |
| CCR086 | | | |
| Methanol | 97 | (+) 0-20% (-) 70-100% | non-lipid epitope |
| Neuraminidase | 89 | (+) 0-40% (-) 88-100% | non-sialic acid epitope |
| Periodate | 81 | (+) 0-30% (-) >75% | non-carbohydrate epitope |
| Heat | 31 | (+) 0-6% (-) ≧50% | inconclusive |
| 6M Guanidine | 85 | (+) ≦20% (-) ≧70% | non-conformatio-nal protein epitope |
| 8M Urea | >100 | (+) ≦30% (-) ≧75% | non-conformatio-nal protein epitope |

EXAMPLE 13

In Vivo Tumor Localization in Nude Mice

A 48-hour biodistribution study was conducted on six T183 tumor-bearing nude mice injected with 10 $\mu$Ci of $^{111}$In-labelled CCR086 at a dose of 1 $\mu$g. Organs and fluids identified in Fig. 2, were extracted, weighed if appropriate and measured for radioactivity. The results of this study are provided in Figure 2. As shown in Fig. 2, about 31% of the labeled CCR086 localized at the tumor site.

EXAMPLE 14

Clinical Investigations

In one study, fourteen patients with 23 known lesions of colon adenocarcinoma were infused intravenously with 5 mCi of $^{111}$In-labeled CCR086 in 2 mg antibody mixed with 3 mg or 17 mg unlabeled CCR086 antibody. Planar imaging or SPECT was performed on days 3, 5 or 6 post infusion. At 5 mg total dose of CCR086, 5 sites were imaged out of 8 known sites (63% detected). At a dose of 20 mg total antibody, 13 sites were imaged out of 15 known (87% detected). In summary, the multi-site imaging protocol conducted on these patients resulted in the detection of 78% of known lesions (18/23), thereby indicating the possible usefulness of CCR086 as a non-CEA marker for targeting colon cancer.

The effectiveness of localizing colorectal carcinoma in vivo with labeled CCR086 was further confirmed in a study involving eight patients with known colorectal cancer. Purified $^{111}$In-labeled CCR086 prepared as described in Example 2 was used to detect the carcinoma in situ. Each patient received 5.5 mCi of $^{111}$In-labeled CCR086 infused over one hour, with six patients receiving a dose of 20 mg and two receiving a dose of 5 mg. Planar imaging and single photon emission computed tomography (SPECT) were usually performed on day 3 and day 7-10 post infusion. The tumor sites were confirmed surgically in four patients and by intercurrent conventional diagnostic modalities in the other four patients.

The results of this clinical investigation, provided in Table 15 below, indicate that $^{111}$In-labeled CCR086 antibody is about 85% effective in localizing colorectal carcinoma in situ. In addition, the procedure was well-tolerated by all patients, with no toxicity observed.

TABLE 15

| In Vivo Detection of Tumor with $^{111}$In-labeled CCR086 | | |
|---|---|---|
| Tumor Sites | | Tumors detected/ Tumors Confirmed |
| 20 mg dose : | | |
| liver metastases | | 5/5 |
| bowel lesions | | 2/2 |
| lung metastases | | 1/2 |
| lymph node metastases | | 2/2 |
| bladder invasion by rectal tumor | | 1/2 |
| | Total | 11/12 |
| 5 mg dose : | | |
| liver lesions | | 1/2 |
| | Total | 1/2 |

In a third in vivo study involving six patients with histologically proven metastatic colon cancer, labeled CCR086 antibody detected the presence or absence of colorectal carcinoma. Each patient received about 5 mCi of $^{111}$In-labeled CCR086 at a dose of 1 mg and either 4 or 19 mg of unlabeled CCR086 antibody via i.v. infusion over a 2 hour period as shown in Table 16. Label integrity of 95-100% was determined by procedures well-known in the art. Blood, biochemical, immunological and urine specimens were taken before infusion and on days 1 and 3 post infusion. After infusion, pharmacokinetic data were accumulated over the first 3 days for blood, and over 2 days for urine. $^{111}$In-labeled CCR086 was determined to have a mean effective half-life of 65 hours, with a mean whole body 48 hour retention of 85-97%. Imaging by planar alone or by planar and SPECT was performed over the chest, pelvic and abdominal areas for 7.5 minutes per image. Table 17 compares the sites of localization detected by labeled CCR086 and by sulfur colloid images.

TABLE 16

| Monoclonal Antibody CCR086 Dosage and Label Integrity | | | |
|---|---|---|---|
| | Quantity Administered | | |
| Patient No. | mg | mCi | % Label |
| 1 | 5 | 5.29 | 100.0 |
| 2 | 20 | 5.39 | 95.9 |
| 3 | 20 | 4.99 | 95.0 |
| 4 | 20 | 5.00 | 99.8 |
| 5 | 20 | 5.13 | 98.9 |
| 6 | 20 | 5.48 | 98.8 |

TABLE 17

| Monoclonal Antibody CCR086 Sites of Localization | | |
|---|---|---|
| Patient No. | Site | Agreement With Sulfur Colloid Scan |
| 1 | Both lobes of liver on subtraction | yes |
| 2 | Numerous areas of liver on subtraction. Right upper chest (over right clavicle, sternoclavicular area). | yes |
| 3 | Multiple defects in liver | yes |
| 4 | Negative study - liver<br>Negative for tumor in lung | negative study |
| 5 | Abdominal carcinomatosis<br>Presacral lesion<br>Bilateral pulmonary metastasis | negative study |
| 6 | Liver<br>Focus in mid-abdomen | yes |

In a further clinical study, six patients with proven colorectal cancer were infused with labelled CCR086 according to the protocol set forth in the third study. All known lesions were detected by labeled CCR086 with the exception of three lesions of less than 1 cm in one patient. In addition, previously unknown lesions in two patients were detected by labelled CCR086. The results of this study were confirmed histologically.

The foregoing description of the invention is exemplary for purposes of illustration and explanation. It will be apparent to those skilled in the art that changes and modifications will be possible without departing from the spirit and scope of the invention. It is intended that the following claims be interpreted to embrace all such changes and modifications.

## Claims

1. An epitope reactive with monoclonal antibody CCR086.

2. An eptiope as claimed in Claim 1, said epitope being expressed on a tumor-associated glycoprotein antigen, said antigen having a molecular weight within the range of about 600 Kd to about 1,000 Kd and isoelectric points within ranges of about 4.3 to about 4.5 and about 4.8 to about 5.0.

3. An epitope, as claimed in Claim 1 or 2, wherein said antigen isoelectric points are about 4.4 and about 4.9.

4. An epitope, as claimed in any one of Claims 1 to 3, wherein said antigen is expressed by colorectal carinoma.

5. An antibody having specific reactivity with an epitope, as defined in any one of Claims 1 to 4.

6. The antibody of Claim 5 wherein said antibody is a monoclonal antibody.

7. An antibody as claimed in Claim 6 which is an IgG$_1$ antibody.

8. Monoclonal antibody CCR086.

9. An antibody, as claimed in any one of Claims 5 to 8, which is labeled with a therapeutic agent.

10. A hybridoma cell line producing an antibody having specificity for an epitope reactive with monoclonal antibody CCR086.

11. A hybridoma cell line as claimed in Claim 10, said epitope being expressed on a tumor-associated glycoprotein antigen, said antigen having a molecular weight in a range of about 600 Kd to about 1,000 Kd and isoelectric points in ranges of about 4.3 about 4.5 and about 4.8 to about 5.0 and present on human colorectal carcinoma.

12. The hybridoma cell line of Claims 10 or 11, wherein said hybridoma cell line is ATCC No. HB 10051.

13. An antibody, as claimed in any one of claims 5 to 9, for use in diagnosis or therapy.

14. Monoclonal antibody CCR086 for use in diagnosis or therapy.

15. A method for in vitro detection of cancer in a patient comprising the steps of: contacting a fluid sample obtained from said patient with at least one antibody as claimed in any one of claims 5 to 8, and determining binding of said antibody to antigenic components of said fluid sample.

16. The method of Claim 15 wherein said antibody is a monoclonal antibody.

17. The method of Claim 16 wherein said monoclonal antibody is CCR086.

18. The method of Claim 17 wherein a two-site immunometric assay employing a second antibody is used to determine the binding of said antibodies to antigenic components of said fluid sample, wherein the antibody of Claim 15 and the second antibody bind non-interfering determinants of said antigen.

19. A pharmaceutical formulation comprising an antibody as claimed in any one of Claims 5 to 9, associated with a pharmaceutically-acceptable carrier, diluent or excipient therefor.

20. A formulation as claimed in Claim 19, wherein said antibody is a monoclonal antibody.

21. A formulation of Claim 20, wherein said monoclonal antibody is CCR086.

Claims for the following Contracting State : GR

1. An epitope reactive with monoclonal antibody CCR086.

2. An eptiope as claimed in Claim 1, said epitope being expressed on a tumor-associated glycoprotein antigen, said antigen having a molecular weight within the range of about 600 Kd to about 1,000 Kd and isoelectric points within ranges of about 4.3 to about 4.5 and about 4.8 to about 5.0.

3. An epitope, as claimed in Claim 1 or 2, wherein said antigen isoelectric points are about 4.4 and about 4.9.

4. An epitope, as claimed in any one of Claims 1 to 3, wherein said antigen is expressed by colorectal carinoma.

5. A process for preparing an antibody which comprises culturing a hybridoma cell line producing an antibody having specificity for an epitope reactive with monoclonal antibody CCR086, and recovering the antibody produced.

6. A process as claimed in Claim 5 in which the antibody is an IgG$_1$ antibody.

7. A process as claimed in Claims 5 or 6 for preparing monoclonal antibody CCR086.

8. A process for preparing an antibody, as claimed in any one of Claims 5 to 7, which additionally

25

comprises labeling the antibody with a therapeutic agent.

9. A hybridoma cell line producing an antibody having specificity for an epitope reactive with monoclonal antibody CCR086.

10. A hybridoma cell line as claimed in Claim 9, said epitope being expressed on a tumor-associated glycoprotein antigen, said antigen having a molecular weight in a range of about 600 Kd to about 1,000 Kd and isoelectric points in ranges of about 4.3 about 4.5 and about 4.8 to about 5.0 and present on human colorectal carcinoma.

11. The hybridoma cell line of Claim 9 or 10, wherein said hybridoma cell line is ATCC No. HB 10051.

12. A method for in vitro detection of cancer in a patient comprising the steps of: contacting a fluid sample obtained from said patient with at least one antibody as defined in any one of claims 5 to 7, and determining binding of said antibody to antigenic components of said fluid sample.

13. The method of Claim 12 wherein the antibody is CCR086.

14. The method of Claim 13 wherein a two-site immunometric assay employing a second antibody is used to determine the binding of said antibodies to antigenic components of said fluid sample, wherein the antibody of Claim 5 to 7 and the second antibody bind non-interfering determinants of said antigen.

15. A pharmaceutical formulation comprising an antibody as claimed in any one of Claims 5 to 8, associated with a pharmaceutically-acceptable carrier, diluent or excipient therefor.

16. A formulation of Claim 15, wherein said monoclonal antibody is CCR086.

17. A process for preparing a formulation, which comprises admixing an antibody as defined in any one of Claims 5 to 8 with a pharmaceutically-acceptable carrier, diluent or excipient.


Claims for the following Contracting State : ES


1. A process for preparing an antibody which comprises culturing a hybridoma cell line producing an antibody having specificity for an epitope reactive with monoclonal antibody CCR086, and recovering the antibody produced.

2. A process as claimed in Claim 1 in which the antibody is an IgG$_1$ antibody.

3. A process as claimed in Claims 1 or 2 for preparing monoclonal antibody CCR086.

4. A process for preparing an antibody, as claimed in any one of Claims 1 to 3, which additionally comprises labeling the antibody with a therapeutic agent.

5. A process as any one of Claims 1 to 4, in which said epitope is expressed on a tumor-associated glycoprotein antigen, said antigen having a molecular weight within the range of about 600 Kd to about 1,000 Kd and isoelectric points within ranges of about 4.3 to about 4.5 and about 4.8 to about 5.0.

6. A process as claimed in Claim 5, wherein said antigen isoelectric points are about 4.4 and about 4.9.

7. A process as claimed in Claim 6 wherein said antigen is expressed by colorectal carinoma.

8. A process as claimed in Claim 7 in which the hybridoma cell line is ATCC No. HB 10051.

9. A process for preparing a formulation, which comprises admixing an antibody prepared according to any one of Claims 1 to 8 with a pharmaceutically-acceptable carrier, diluent or excipient.

FIG. 1

EP 0 397 419 A2

FIG. 2